# EUROPEAN PATENT SPECIFICATION

(11) **EP 2 349 342 B1**
(45) Date of publication and mention of the grant of the patent: **27.06.2018**
(21) Application number: 09752930.9
(22) Date of filing: 16.10.2009
(51) Int. Cl.: A61K 47/60, A61K 47/61, A61K 38/37, A61P 7/02, A61P 7/04

(54) **MODIFIED BLOOD FACTORS COMPRISING A LOW DEGREE OF WATER SOLUBLE POLYMER**
MODIFIZIERTE BLUTFAKTOREN MIT GERINGEM GRAD AN WASSERLÖSLICHEM POLYMER
FACTEURS SANGUINS MODIFIÉS RENFERMANT UN FAIBLE DEGRÉ DE POLYMÈRE SOLUBLE DANS L'EAU

(30) Priority: 17.10.2008 US 106424 P
(43) Date of publication of application: 03.08.2011
(73) Proprietor: Baxalta GmbH, 6300 Zug (CH); Baxalta Incorporated, Bannockburn, IL 60015 (US)
(72) Inventor: TURECEK, Peter, A-3400 Klosterneuburg (AT); SIEKMANN, Juergen, A-1210 Vienna (AT); ROTTENSTEINER, Hanspeter, A-1020 Vienna (AT)
(74) Representative: Hoffmann Eitle
(86) International application number: PCT/US2009/061023
(87) International publication number: WO 2010/045568

(56) References cited:
- WO-A1-94/29370
- WO-A1-2004/000366
- WO-A1-2008/074032
- WO-A2-2006/005058
- WO-A2-2006/114105
- WO-A2-2006/127896
- WO-A2-2008/082669
- WO-A2-2010/014708
- US-A1- 2004 235 734
- US-A1- 2006 115 876
- US-A1- 2007 244 301
- US-A1- 2007 254 840
- RÖSTIN J ET AL: "B-Domain Deleted Recombinant Coagulation Factor VIII Modified with Monomethoxy Polyethylene Glycol" BIOCONJUGATE CHEMISTRY, ACS, WASHINGTON, DC, US, vol. 11, 4 April 2000 (2000-04-04), pages 387-396, XP002249353 ISSN: 1043-1802
- SAENKO E L ET AL: "Strategies towards a longer acting factor VIII", HAEMOPHILIA, BLACKWELL SCIENCE, OXFORD, GB, vol. 12, no. SUPPL. 3, 1 January 2006 (2006-01-01), pages 42-51, XP002434557, ISSN: 1351-8216, DOI: 10.1111/J.1365-2516.2006.01260.X
- FERNANDES A I ET AL: "Polysialylated asparaginase: preparation, activity and pharmacokinetics", BIOCHIMICA ET BIOPHYSICA ACTA. PROTEIN STRUCTURE AND MOLECULAR ENZYMOLOGY, ELSEVIER, AMSTERDAM; NL, vol. 1341, no. 1, 15 August 1997 (1997-08-15), pages 26-34, XP027382387, ISSN: 0167-4838 [retrieved on 1997-08-15]

## Description

### FIELD OF THE INVENTION

The present invention relates, in general, to materials and methods for the preparation of modified blood factors which comprise low levels of water soluble polymer molecules but exhibit biological activity similar to molecules having a higher number of water soluble polymer moieties.

### BACKGROUND OF THE INVENTION

Blood coagulation is a complex process including the sequential interaction of a series of components, in particular of fibrinogen, Factor II, Factor V, Factor VII, Factor VIIa, Factor VIII, Factor IX, Factor X, Factor XI, Factor XII and von Willebrand Factor. The loss of one of these components or the inhibition of its functionality may cause either an increased tendency of blood coagulation or an inability to clot, either of which may be life-threatening in some patients.

Factor VIII is a cofactor for Factor IXa which converts Factor X to Factor Xa in the cascade of reactions leading to blood coagulation. A deficiency in the amount of Factor VIII activity in the blood results in the clotting disorder hemophilia A, an inherited condition primarily affecting males. Hemophilia A is currently treated with therapeutic preparations of Factor VIII derived from human plasma or manufactured using recombinant DNA technology. Such preparations are administered either in response to a bleeding episode or at frequent, regular intervals to prevent uncontrolled bleeding (prophylaxis).

Von Willebrand Factor (VWF) circulates in plasma complexed with Factor VIII, which stabilizes the Factor VIII protein and protects it from proteolytic degradation. Due to its function in platelet aggregation, VWF also directly interferes in blood coagulation. Von Willebrand deficiency (VWD) (also known as von Willebrand syndrome) results from either a deficiency or overexpression of VWF. Deficiency of VWF results in a disease similar to hemophilia due to the rapid degradation of Factor VIII lacking VWF cofactor.

In treatment of Hemophilia A and von Willebrand syndrome there have been a number of attempts to treat patients with purified Factor VII, VWF or Factor VIII/VWF-complex. The development of antibodies, however, against the administered exogenous protein has been shown to decrease the efficacy of treatment and presents a challenge to treatment of these patients. For example, anti-FVIII antibodies are especially prevalent in patients with severe and moderately severe hemophilia, which develop anti-FVIII antibodies at a frequency of 50% (Gilles et al., Blood 82:2452-61, 1993; Lacroix-Desmazes et al., J Immunol. 177:1355-63, 2006). Administration of lower doses of therapeutic protein which have greater efficacy in vivo would help reduce or limit the occurrence of antibodies against the administered blood factor.

The pharmacokinetic and immunological properties of therapeutic proteins can be improved by conjugation with a water soluble polymer such as polyethyleneglycol (PEG). In particular, binding a physiologically active protein to a physiologically acceptable polymer molecule can substantially prolong its *in vivo* half-life. PEGylation of molecules can also lead to increased resistance of drugs to enzymatic degradation, reduced dosing frequency, decreased immunogenicity, increased physical and thermal stability, increased solubility, increased liquid stability, and reduced aggregation.

U.S. Patent 4,970,300 describes that the conjugation of a polymer molecule (dextran) to Factor VIII (FVIII) results in a FVIII protein being activatable by thrombin, and having a substantially decreased antigenicity and immunoreactivity and a substantially increased *in vivo* retention time in the bloodstream of a mammal. International patent application WO 94/15625 describes that conjugating FVIII to a physiologically acceptable polymer molecule improves the *in vivo* function of FVIII (i) by increasing its resistance to *in vivo* hydrolysis and thus prolonging its activity after administration, (ii) by significantly prolonging its circulating life *in vivo* over unmodified protein, and (iii) by increasing its absorption time into the blood stream. U.S. Patent 6,037,452 describes FVIII and Factor IX (FIX) conjugates, where the protein is covalently bound to a poly(alkylene oxide) through carbonyl-groups in the protein. Further, improving the *in vivo* function of FIX by conjugation to physiologically acceptable polymer molecules, in particular poly(ethylene glycol) ("PEG"), has been described in international patent publication WO 94/29370. A PEGylated FVIII that retains specific activity was disclosed in International Patent Publication WO/2007/126808.

The conjugation of water soluble polymer to an active agent such as a protein can be performed by preparing stable polymer-protein conjugates or polymer-protein conjugates in which the water soluble polymer is attached to the protein via releasable covalent bonds (pro-drug concept), i.e., a hydrolyzable, degradable or releasable linker. For example, a releasable PEG moiety has been developed using a 9-fluorenylmethoxycarbonyl (FMOC) conjugation system containing two PEG chains (Nektar Inc., Huntsville AL). In addition an N-hydroxysuccinimide ester (NHS) group, which is useful for the chemical modification of lysine residues of the protein, may be linked to the fluorene ring system via the methoxycarbonyl group to generate the releasable PEG moiety. International Patent Publication WO 2008/082669 (incorporated herein by reference) describes a series of PEGylated recombinant FVIII variants based on the releasable PEG concept.

A chemical process leading to a relatively high degree of modification and water soluble polymer content on the therapeutic protein is not economical due to the high amounts of reagents required for preparation. In addition, high degrees of water soluble polymer, such as PEG, lead to an increased toxicological or immunological risk due to high amounts of the polymer and the linker.

Thus, there remains a need in the art for therapeutic compositions comprising water soluble polymers which improve the half-life and stability of a therapeutic protein without the resultant toxicity or immunological effects.

### SUMMARY OF THE INVENTION

The present specification is directed to materials and methods for generating blood factor variants comprising low amounts of a water soluble polymer. The invention provides a chemically modified blood factor molecule comprising a recombinant blood factor, i.e. FVIII, and 1 or 2 water soluble polymer moieties per blood factor molecule, wherein the water soluble polymer is polysialic acid (PSA) and wherein the PSA moieties are attached to the FVIII molecule through reductive amination.

In a related embodiment, the modified blood factor comprises 2 water soluble polymer moieties per blood factor molecule. In still another embodiment, the modified blood factor comprises 1 water soluble polymer per blood factor molecule.

It is contemplated that the water soluble polymer moiety is attached to the blood factor molecule through a stable linker or through a releasable or degradable linker. In one embodiment, the releasable linker is a hydrolyzable linker.

The water soluble polymer polysialic acid (PSA).

In a reference aspect, the water soluble polymer is polyethylene glycol (PEG). It is further contemplated that the PEG molecule is a linear PEG moiety or a branched PEG moiety.

In another reference aspect, the PEG has a molecular weight between 3 kD and 200 kD. In a related embodiment the average molecular weight of the PEG will range from about 3 to 200 kiloDalton ("kDa"), from about 5 kDa to about 120 kDa, from about 10 kDa to about 100 kDa, from about 20 kDa to about 50 kDa, from about 10 kDa to about 25 kDa, from about 5 kDa to about 50 kDa, or from about 5 kDa to about 10 kDa.

The invention contemplates that the modified blood factor is Factor VIII. In a further embodiment, blood factor molecule is human.

In a reference aspect, the modified blood factor comprises at least 4 and less than 10 PEG moieties per Factor VIII molecule. In another reference aspect the modified blood factor comprises between 4 and 8 PEG moieties, inclusive, per Factor VIII molecule. In still another reference aspect the modified blood factor comprises between 1 and 4 PEG moieties, inclusive, per Factor VIII molecule. In still another reference aspect the modified blood factor comprises between 4 and 6 PEG moieties, inclusive, per Factor VIII molecule. In still another reference aspect the modified blood factor comprises 1 or 2 PEG moieties per Factor VIII molecule.

The modified blood factor comprises 1 or 2 PSA moieties per Factor VIII molecule.
In another embodiment the modified blood factor comprises 1 or 2 PSA moieties per Factor VIII molecule, wherein the polymers are connected via a stable, releasable or hydrolysable linker.

In another reference aspect, the modified blood factor comprises between 1 and 6 water soluble polymer moieties, inclusive, per FVIIa molecule. In a related reference aspect, the modified blood factor comprises 1 or 2 water soluble polymer moieties per FVIIa molecule. In a reference aspect, the water soluble polymer is selected from the group consisting of PEG or PSA. In one reference aspect, the polymers are connected via a stable, releasable or hydrolyzable linker.

In yet another reference aspect, the modified blood factor comprises between 1 and 6 water soluble polymer moieties, inclusive, per FVIIa molecule. In a still further reference aspect, the modified blood factor comprises 1 or 2 water soluble polymer moieties per FIX molecule. In certain reference aspects, the water soluble polymer is selected from the group consisting of PEG or PSA. In one reference aspect, the polymers are connected via a stable, releasable or hydrolyzable linker.

In a further aspect, the invention provides a pharmaceutical composition comprising the modified blood factor as described herein.

In another reference aspect, the specification contemplates a method of making a modified blood factor molecule having a low number of water soluble polymer conjugated to the blood factor molecule comprising, contacting the blood factor molecule with a molar excess of water soluble polymer less than or equal to 30 M excess water soluble polymer to blood factor molecule under conditions that permit attachment of at least 1 and less than 10 water soluble polymers to the blood factor molecule.

In one embodiment, the molar excess of water soluble polymer is between 2 M excess and 30 M excess. In another embodiment, the molar excess of water soluble polymer is between 10 M and 25 M excess. In still another embodiment, the molar excess of water soluble polymer is about 2 M, about 5 M, about 10 M, about 15 M, about 20 M, about 25 M, or about 30 M excess.

In yet another aspect, the specification provides a method of treating a subject suffering from a blood clotting disorder comprising administering to the patient a therapeutically effective amount of a modified blood factor as described herein.

In one embodiment, the blood clotting disorder is selected from the group consisting of hemophilia A, hemophilia B, von Willebrand syndrome, Factor X deficiency, Factor VII deficiency, Alexander's disease, Rosenthal syndrome (hemophilia C) and Factor XIII deficiency.

In a related embodiment, the blood clotting disorder is hemophilia A and the modified blood factor comprises a Factor VIII molecule.

In a further embodiment, the water soluble polymer for use in the methods is a water soluble polymer as described above.

In still another reference aspect, the blood factor for use in the methods is selected from the group consisting of Factor II, Factor III, Factor V, Factor VII. Factor VIIa, Factor VIII, Factor IX , Factor X, Factor XI, von Willebrand Factor and fibrinogen. In one embodiment, the blood factor molecule is Factor VIII. In still another embodiment the blood factor is FVIIa. In a further embodiment, the blood factor is FIX. In a further embodiment, blood factor molecule is human.

### BRIEF DESCRIPTION OF THE DRAWINGS

Figure 1 shows an SDS-PAGE gel of low PEGylated Factor VIII prepared using a low molar excess of PEG. Figure 1A: stained with anti-FVIII antibody, Figure 1B: stained with anti-PEG antibody.
Figure 2 depicts the pharmacokinetic profile of low PEGylated FVIII as detected in vivo in a FVIII deficient mouse model. The data are dose adjusted to 200 U/kg.
Figure 3 illustrates the comparison of the degree of PEGylation of the FVIII correlated with the AUC (Figure 3A) of the PEGylated protein, with the half life (HL, Figure 3B) and with the mean resistance time (MRT, Figure 3C) as detected in vivo in a FVII deficient mouse model.
Figure 4 depicts the pharmocokinetic profile of low Polysialylated rFVIII as detected in vivo in a FVIII deficient mouse model. The data are adjusted to 200 U/kg.

### DETAILED DESCRIPTION OF THE INVENTION

The present specification is directed to materials and methods for generating blood factor variants comprising low amounts of a water soluble polymer wherein the amount of the water soluble polymer, such as PEG, is sufficient to prolong half life of the molecule. It is contemplated that the modified blood factor comprising a low amount of water soluble polymer demonstrates reduced toxicity compared to blood factor molecules prepared using standard preparation protocols.

Unless otherwise defined, all technical and scientific terms used herein have the same meaning as commonly understood by one of ordinary skill in the art to which this invention belongs. The following references provide one of skill with a general definition of many of the terms used in this invention: Singleton, et al., DICTIONARY OF MICROBIOLOGY AND MOLECULAR BIOLOGY (2d ed. 1994); THE CAMBRIDGE DICTIONARY OF SCIENCE AND TECHNOLOGY (Walker ed., 1988); THE GLOSSARY OF GENETICS, 5TH ED., R. Rieger, et al. (eds.), Springer Verlag (1991); and Hale and Marham, THE HARPER COLLINS DICTIONARY OF BIOLOGY (1991).

It is noted here that, as used in this specification and the appended claims, the singular forms "a," "an," and "the" include plural reference unless the context clearly dictates otherwise.

As used herein, the following terms have the meanings ascribed to them unless specified otherwise

The term "modified blood factor" refers to a blood factor having one or more modifications such as conjugation to a water-soluble polymer, conjugation to additional carbohydrate moieties or otherwise modified from the native or wild-type blood clotting factor. A "blood factor" or "blood clotting factor" as used herein refers to proteins involved in blood clotting in a subject, including those involved in the clotting cascade. Blood factors, include, but are not limited to, Factor II, Factor III, Factor V, Factor VII, Factor VIIa, Factor VIII, Factor IX , Factor X, Factor XI, von Willebrand Factor and fibrinogen.

The term "protein" refers to any protein, protein complex or polypeptide, including recombinant proteins, protein complexes and polypeptides composed of amino acid residues linked via peptide bonds. Proteins are obtained by isolation from in vivo sources (i.e., naturally-occurring), by synthetic methods, or by recombinant DNA technology. Synthetic polypeptides are synthesized, for example, using an automated polypeptide synthesizer. A recombinant protein used according to the present invention is produced by any method known in the art as described herein below. In one embodiment, the protein is a physiologically active protein, including a therapeutic protein or a biologically active derivative thereof. The term "biologically active derivative" refers to a derivative of a protein having substantially the same functional and/or biological properties of said protein. The term "protein" typically refers to large polypeptides. The term "peptide" typically refers to short polypeptides. Regardless of the distinction, as used herein, polypeptide, protein and peptide are used interchangeably.

A "fragment" of a polypeptide refers to any portion of the polypeptide smaller than the full-length polypeptide or protein expression product. Fragments are, in one aspect, deletion analogs of the full-length polypeptide wherein one or more amino acid residues have been removed from the amino terminus and/or the carboxy terminus of the full-length polypeptide. Accordingly, "fragments" are a subset of deletion analogs described below.

An "analogue," "analog" or "derivative" is a compound, e.g., a peptide, refers to a polypeptide substantially similar in structure and having the same biological activity, albeit in certain instances to a differing degree, to a naturally-occurring molecule. Analogs differ in the composition of their amino acid sequences compared to the naturally-occurring polypeptide from which the analog is derived, based on one or more mutations involving (i) deletion of one or more amino acid residues at one or more termini of the polypeptide and/or one or more internal regions of the naturally-occurring polypeptide sequence, (ii) insertion or addition of one or more amino acids at one or more termini (typically an "addition" analog) of the polypeptide and/or one or more internal regions (typically an "insertion" analog) of the naturally-occurring polypeptide sequence or (iii) substitution of one or more amino acids for other amino acids in the naturally-occurring polypeptide sequence.

In one aspect, an analog exhibits about 70% sequence similarity but less than 100% sequence similarity with a given compound, e.g., a peptide. Such analogs or derivatives are, in one aspect, comprised of non-naturally occurring amino acid residues, including by way of example and not limitation, homoarginine, ornithine, penicillamine, and norvaline, as well as naturally occurring amino acid residues. Such analogs or derivatives are, in another aspect, composed of one or a plurality of D-amino acid residues, or contain non-peptide interlinkages between two or more amino acid residues. The term "derived from" as used herein refers to a polypeptide or peptide sequence that is a modification (including amino acid substitution or deletion) of a wild-type or naturally-occurring polypeptide or peptide sequence and has one or more amino acid substitutions, additions or deletions, such that the derivative sequence shares about 70% but less than 100% sequence similarity to the wild-type or naturally-occurring sequence. In one embodiment, the derivative may be a fragment of a polypeptide, wherein the fragment is substantially homologous (i.e., at least 70%, at least 75%, at least 80%, at least 85%, at least 90%, or at least 95% homologous) over a length of at least 5, 10, 15, 20, 25, 30, 35, 40, 45 or 50 amino acids of the wild-type polypeptide.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection. One example of a useful algorithm is PILEUP, which uses a simplification of the progressive alignment method of Feng & Doolittle, J. Mol. Evol. 35:351-360 (1987) and is similar to the method described by Higgins & Sharp, CABIOS 5:151-153 (1989). Another algorithm useful for generating multiple alignments of sequences is Clustal W (Thompson, et al., Nucleic Acids Research 22: 4673-4680 (1994)). An example of algorithm that is suitable for determining percent sequence identity and sequence similarity is the BLAST algorithm (Altschul, et al., J. Mol. Biol. 215:403-410 (1990); Henikoff & Henikoff, Proc. Natl. Acad. Sci. USA 89:10915 (1989); Karlin & Altschul, Proc. Natl. Acad. Sci. USA 90:5873-5787 (1993)). Software for performing BLAST analyses is publicly available through the National Center for Biotechnology Information.

Substitutions are conservative or non-conservative based on the physico-chemical or functional relatedness of the amino acid that is being replaced and the amino acid replacing it. Substitutions of this type are well known in the art. Alternatively, the specification embraces substitutions that are also non-conservative. Exemplary conservative substitutions are described in Lehninger, [Biochemistry, 2nd Edition; Worth Publishers, Inc., New York (1975), pp.71-77] and set out below.

**CONSERVATIVE SUBSTITUTIONS**

| **SIDE CHAIN CHARACTERISTIC** | **AMINO ACID** |
|---|---|
| Non-polar (hydrophobic): | |
| A. Aliphatic | A L I V P |
| B. Aromatic | F W |
| C. Sulfur-containing | M |
| D. Borderline | G |
| Uncharged-polar: | |
| A. Hydroxyl | S T Y |
| B. Amides | N Q |
| C. Sulfhydryl | C |
| D. Borderline | G |
| Positively charged (basic) | K R H |
| Negatively charged (acidic) | D E |

Alternatively, exemplary conservative substitutions are set out immediately below.

**CONSERVATIVE SUBSTITUTIONS II**

| **ORIGINAL RESIDUE** | **EXEMPLARY SUBSTITUTION** |
|---|---|
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| Gln (Q) | Asn |
| Glu (E) | Asp |
| His (H) | Asn, Gln, Lys, Arg |
| Ile (I) | Leu, Val, Met, Ala, Phe, |
| Leu (L) | Ile, Val, Met, Ala, Phe |
| Lys (K) | Arg, Gln, Asn |
| Met (M) | Leu, Phe, Ile |
| Phe (F) | Leu, Val, Ile, Ala |
| Pro (P) | Gly |
| Ser (S) | Thr |
| Thr (T) | Ser |
| Trp (W) | Tyr |
| Tyr (Y) | Trp, Phe, Thr, Ser |
| Val (V) | Ile, Leu, Met, Phe, Ala |

The term "variant" refers to a protein or analog thereof that is modified to comprise additional chemical moieties not normally a part of the molecule. Such moieties improve, in various aspects, including but not limited to, the molecule's solubility, absorption and biological half-life. The moieties may alternatively decrease the toxicity of the molecule and eliminate or attenuate any undesirable side effect of the molecule, etc. Moieties capable of mediating such effects are disclosed in Remington's Pharmaceutical Sciences (1980). Procedures for coupling such moieties to a molecule are well known in the art. In certain aspects, without limitation, variants are polypeptides that are modified by glycosylation, PEGylation, or polysialylation.

The term "naturally-occurring," as applied to a protein or polypeptide, refers a protein is found in nature. For example, a polypeptide or polynucleotide sequence that is present in an organism (including viruses) that is isolated from a source in nature and which has not been intentionally modified by man in the laboratory is naturally-occurring. The terms "naturally-occurring" and "wild-type" are used interchangeably throughout.

The term "plasma-derived," as applied to a protein or polypeptide, refers to a naturally-occurring polypeptide or fragment thereof that is found in blood plasma or serum of a subject.

The term "water soluble polymer" refers to polymer molecules which are substantially soluble in aqueous solution or are present in the form of a suspension and have substantially no negative impact to mammals upon administration of a protein conjugated to said polymer in a pharmaceutically effective amount and can be regarded as biocompatible. In one embodiment, physiologically acceptable molecules comprise from about 2 to about 300 repeating units. Exemplary water soluble polymers include, but are not limited to, poly(alkylene glycols) such as polyethylene glycol (PEG), poly(propylene glycol) ("PPG"), copolymers of ethylene glycol and propylene glycol and the like, poly(oxyethylated polyol), poly(olefinic alcohol), poly(vinylpyrrolidone), poly(hydroxyalkylmethacrylamide), poly(hydroxyalkylmethacrylate), poly(saccharides), poly(α-hydroxy acid), poly(vinyl alcohol), polyphosphasphazene, polyoxazoline, poly(N-acryloylmorpholine), poly(alkylene oxide) polymers, poly(maleic acid), poly(DL-alanine), polysaccharides, such as carboxymethylcellulose, dextran, starch or starch derivatives, hyaluronic acid and chitin, poly(meth)acrylates, and combinations of any of the foregoing.

The water soluble polymer molecule is not limited to a particular structure and, in certain aspects, is branched or multi-armed, dendritic, or with degradable, releasable or hydrolyzable linkages. Moreover, the internal structure of the polymer molecule are, in still other aspects, are organized in any number of different patterns and are selected from the group consisting of, without limitation, homopolymer, alternating copolymer, random copolymer, block copolymer, alternating tripolymer, random tripolymer, and block tripolymer.

The term "PEGylated" refers to a protein, protein complex or polypeptide bound to one or more PEG moieties. The term "PEGylation" as used herein refers to the process of binding one or more PEGs to a protein. The molecular weight of PEG may be in the range of from 2 to 200 kDa, from 5 to 120 kDa, from 10 to 100 kDa, from 20 to 50 kDa, from 10 to 25 kDa, from 5 kDa to 10 kDa, or from 2 kDa to 5 kDa.

The term "polysialylated" refers to a protein, protein complex or polypeptide bound to one or more polysialic acid (PSA) moieties. The term "polysialylation" as used herein refers to the process of binding one or more PSA moieties to a protein. In one embodiment, the molecular weight of said PSA is in the range of from 2 to 80 kDa, from 5 to 60 kDa, from 10 to 40 kDa or from 15 to 25 kDa.

The term "linker" refers to a molecular fragment that links the water soluble polymer to a biologically active molecule. The fragment typically has two functional groups that can be coupled to or activated to react with another linker or directly with the biologically active nucleophile. As an example, ω-aminoalkanoic acid such as lysine is commonly used. In the present invention, linkers includes stable, releasable, degradable and hydrolyzable linkers.

The term "pharmaceutical composition" refers to a composition suitable for pharmaceutical use in subject animal, including humans and mammals. A pharmaceutical composition comprises a pharmacologically effective amount of a polymer-polypeptide conjugate and also comprises a pharmaceutically acceptable carrier. A pharmaceutical composition encompasses a composition comprising the active ingredient(s), and the inert ingredient(s) that make up the pharmaceutically acceptable carrier, as well as any product which results, directly or indirectly, from combination, complexation or aggregation of any two or more of the ingredients. Accordingly, the pharmaceutical compositions of the present invention encompass any composition made by admixing a compound or conjugate of the present invention and a pharmaceutically acceptable carrier.

The term "pharmaceutically acceptable carrier" includes any and all clinically useful solvents, dispersion media, coatings, antibacterial and antifungal agents, isotonic and absorption delaying agents, buffers, and excipients, such as a phosphate buffered saline solution, 5% aqueous solution of dextrose, and emulsions, such as an oil/water or water/oil emulsion, and various types of wetting agents and/or adjuvants. Suitable pharmaceutical carriers and formulations are described in Remington's Pharmaceutical Sciences, 19th Ed. (Mack Publishing Co., Easton, 1995). Pharmaceutical carriers useful for the composition depend upon the intended mode of administration of the active agent. Typical modes of administration include, but are not limited to, enteral (*e.g*., oral) or parenteral (*e.g*., subcutaneous, intramuscular, intravenous or intraperitoneal injection; or topical, transdermal, or transmucosal administration). A "pharmaceutically acceptable salt" is a salt that can be formulated into a compound or conjugate for pharmaceutical use including, *e.g*., metal salts (sodium, potassium, magnesium, calcium, *etc*.) and salts of ammonia or organic amines.

The term "pharmaceutically acceptable" or "pharmacologically acceptable" is meant a material which is not biologically or otherwise undesirable, *i.e*., the material may be administered to an individual without causing any undesirable biological effects or interacting in a deleterious manner with any of the components of the composition in which it is contained, or when administered using routes well-known in the art, as described below.

The term "blood clotting disorder" or "bleeding disorder" refers to any of several inherited or developed deficiencies in blood clotting factors which lead to the inability of blood to efficiently form clots, and subsequent aberrant bleeding in a subject. Blood clotting disorders include but are not limited to, hemophilia A, hemophilia B, von Willebrand syndrome, Factor X deficiency, Factor VII deficiency, Alexander's disease, Rosenthal syndrome (hemophilia C) and Factor XIII deficiency. Treatment of a blood clotting disorder refers to prophylactic treatment or therapeutic treatment.

"Treatment" refers to prophylactic treatment or therapeutic treatment. A "prophylactic" treatment is a treatment administered to a subject who does not exhibit signs of a disease or exhibits only early signs for the purpose of decreasing the risk of developing pathology. The compounds or conjugates of the invention may be given as a prophylactic treatment to reduce the likelihood of developing a pathology or to minimize the severity of the pathology, if developed. A "therapeutic" treatment is a treatment administered to a subject who exhibits signs or symptoms of pathology for the purpose of diminishing or eliminating those signs or symptoms. The signs or symptoms may be biochemical, cellular, histological, functional, subjective or objective. The compositions of the invention may be given as a therapeutic treatment or for diagnosis.

As used herein, the term "subject" encompasses mammals and non-mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish, and the like. The term does not denote a particular age or gender.

The term "effective amount" means a dosage sufficient to produce a desired result on a health condition, pathology, and disease of a subject or for a diagnostic purpose. The desired result may comprise a subjective or objective improvement in the recipient of the dosage. "Therapeutically effective amount" refers to that amount of an agent effective to produce the intended beneficial effect on health. An appropriate "effective" amount in any individual case may be determined by one of ordinary skill in the art using routine experimentation.

### Proteins and Protein Complexes

Proteins contemplated for use in pharmaceutical compositions include physiologically active blood clotting factors useful for administration to a subject. The blood clotting factor is a protein or any fragment, analog, or variant of such that still retains some, substantially all, or all of the therapeutic or biological activity of the protein. In some embodiments, the blood clotting factor is one that, if not expressed or produced or if substantially reduced in expression or production, would give rise to a disease. In one aspect, the blood clotting factor is derived or obtained from a mammal.

The blood clotting factor conjugated to a water soluble polymer may be a blood clotting factor or fragment thereof possessing a biological activity of the protein, and has an amino acid sequence identical to the amino acid sequence to the corresponding portion of the human or mammalian protein. The blood clotting factor of the conjugate may be a protein native to the species of the human or mammal. The blood clotting factor or fragment thereof, may be substantially homologous (i.e., at least 80%, 85%, 90%, 95%, more preferably 98%, or most preferably 99% identical in amino acid sequence over a length of at least 10, 25, 50, 100, 150, or 200 amino acids, or the entire length of the active agent) to a native sequence of the corresponding human or mammal protein.

### Methods of making a protein

Methods for making recombinant proteins are well-known in the art. Methods of producing cells, including mammalian cells, which express DNA or RNA encoding a recombinant protein are described in U.S. patent numbers 6,048,729, 5,994,129, and 6,063,630.

A nucleic acid construct used to express a polypeptide or fragment, variant or analog thereof is, in one aspect, one which is expressed extrachromosomally (episomally) in the transfected mammalian cell or one which integrates, either randomly or at a pre-selected targeted site through homologous recombination, into the recipient cell's genome. A construct which is expressed extrachromosomally comprises, in addition to polypeptide-encoding sequences, sequences sufficient for expression of the protein in the cells and, optionally, for replication of the construct. It optionally includes a promoter, a polypeptide-encoding DNA sequence and/or a polyadenylation site. The DNA encoding the protein is positioned in the construct in such a manner that its expression is under the control of the promoter. Optionally, the construct contains additional components such as one or more of the following: a splice site, an enhancer sequence, a selectable marker gene under the control of an appropriate promoter, and an amplifiable marker gene under the control of an appropriate promoter.

In those embodiments in which the DNA construct integrates into the cell's genome, it need, in one aspect, include only the polypeptide-encoding nucleic acid sequences. Optionally, the construct includes a promoter and an enhancer sequence, a polyadenylation site or sites, a splice site or sites, nucleic acid sequences which encode a selectable marker or markers, nucleic acid sequences which encode an amplifiable marker and/or DNA homologous to genomic DNA in the recipient cell to target integration of the DNA to a selected site in the genome (targeting DNA or DNA sequences).

### Host cells

Host cells used to produce recombinant proteins are, for example and without limitation, bacterial, yeast, insect, non-mammalian vertebrate, or mammalian cells; the mammalian cells include, but are not limited to, hamster, monkey, chimpanzee, dog, cat, bovine, porcine, mouse, rat, rabbit, sheep and human cells. The host cells are immortalized cells (a cell line) or non-immortalized (primary or secondary) cells and are any of a wide variety of cell types, such as, but not limited to, fibroblasts, keratinocytes, epithelial cells (e.g., mammary epithelial cells, intestinal epithelial cells), ovary cells (e.g., Chinese hamster ovary or CHO cells), endothelial cells, glial cells, neural cells, formed elements of the blood (e.g., lymphocytes, bone marrow cells), muscle cells, hepatocytes and precursors of these somatic cell types. Commonly used host cells include, without limitation: prokaryotic cells such as gram negative or gram positive bacteria, *i.e*., any strain of *E. coli, Bacillus, Streptomyces, Saccharomyces, Salmonella,* and the like; eukaryotic cells such as CHO (Chinese hamster ovary) cells; baby hamster kidney (BHK) cells; human kidney 293 cells; COS-7 cells; insect cells such as D. Mel-2, Sf4, Sf5, Sf9, and Sf21 and High 5; plant cells and various yeast cells such as *Saccharomyces* and *Pichia.*

Host cells containing the polypeptide-encoding DNA or RNA are cultured under conditions appropriate for growth of the cells and expression of the DNA or RNA. Those cells which express the polypeptide are identified, using known methods, and the recombinant protein isolated and purified, using known methods; either with or without amplification of polypeptide production. Identification is carried out, for example and without limitation, through screening genetically modified mammalian cells displaying a phenotype indicative of the presence of DNA or RNA encoding the protein, such as PCR screening, screening by Southern blot analysis, or screening for the expression of the protein. Selection of cells having incorporated protein-encoding DNA is accomplished, for example, by including a selectable marker in the DNA construct and culturing transfected or infected cells containing a selectable marker gene under conditions appropriate for survival of only those cells that express the selectable marker gene. Further amplification of the introduced DNA construct is affected by culturing genetically modified cells under conditions appropriate for amplification (e.g., culturing genetically modified cells containing an amplifiable marker gene in the presence of a concentration of a drug at which only cells containing multiple copies of the amplifiable marker gene can survive).

Recombinant proteins which are physiologically active proteins or therapeutic proteins include, but are not limited to, cytokines, growth factors, blood clotting factors, enzymes, chemokines, soluble cell-surface receptors, cell adhesion molecules, antibodies, hormones, cytoskeletal proteins, matrix proteins, chaperone proteins, structural proteins, metabolic proteins, and other therapeutic proteins known to those of skill in the art.

Exemplary recombinant blood clotting factors which are used as therapeutics include, but are not limited to, Factor II, Factor III, Factor V, Factor VII, Factor VIIa, Factor VIII, Factor IX , Factor X, Factor XI, von Willebrand Factor and fibrinogen. The protein complex may be a complex comprising one or more blood factors.

### Blood Factors

Factor VIII (FVIII) is a blood plasma glycoprotein of about 260 kDa molecular mass produced in the liver of mammals (Genbank Accesion No. NP_000123). It is a critical component of the cascade of coagulation reactions that lead to blood clotting. Within this cascade is a step in which Factor IXa, in conjunction with FVIII, converts Factor X (Genbank Accession No. NP_000495) to an activated form, Factor Xa. FVIII acts as a cofactor at this step, being required with calcium ions and phospholipid for the activity of Factor IXa. The two most common hemophilic disorders are caused by a deficiency of functional FVIII (Hemophilia A, about 80% of all cases) or functional Factor IXa (Hemophilia B or Christmas Factor disease). FVIII circulates, in plasma at a very low concentration and is bound non-covalently to von Willebrand Factor (VWF). During hemostasis, FVIII is separated from VWF and acts as a cofactor for activated Factor IX (FIXa)-mediated Factor X (FX) activation by enhancing the rate of activation in the presence of calcium and phospholipids or cellular membranes.

FVIII is synthesized as a single-chain precursor of approximately 270-330 kD with the domain structure A1-A2-B-A3-C1-C2. When purified from plasma, FVIII is composed of a heavy chain (A1-A2-B) and a light chain (A3-C1-C2). The molecular mass of the light chain is 80 kD whereas, due to proteolysis within the B domain, the heavy chain is in the range of 90-220 kD.

FVIII is also synthesized as a recombinant protein for therapeutic use in bleeding disorders. Various in vitro assays have been devised to determine the potential efficacy of recombinant FVIII (rFVIII) as a therapeutic medicine. These assays mimic the in vivo effects of endogenous FVIII. In vitro thrombin treatment of FVIII results in a rapid increase and subsequent decrease in its procoagulant activity, as measured by in vitro assay. This activation and inactivation coincides with specific limited proteolysis both in the heavy and the light chains, which alter the availability of different binding epitopes in FVIII, e.g., allowing FVIII to dissociate from VWF and bind to a phospholipid surface or altering the binding ability to certain monoclonal antibodies.

Until recently, the standard treatment of Hemophilia A involved frequent infusion of preparations of FVIII concentrates derived from the plasmas of human donors. While this replacement therapy is generally effective, such treatment puts patients at risk for virus-transmissible diseases such as hepatitis and AIDS. Although this risk has been reduced by further purification of FVIII from plasma by immunopurification using monoclonal antibodies, and by inactivating viruses by treatment with either an organic solvent or heat, such preparations have greatly increased the cost of treatment and are not without risk. For these reasons, patients have been treated episodically, rather than prophylactically. A further complication is that about 15% of patients develop inhibitory antibodies to plasma-derived FVIII. Patients with severe haemophilia A with FVIII levels below 1%, are generally on prophylactic therapy with the aim of keeping FVIII above 1% between doses. Taking into account the average half-lives of the various FVIII products in the circulation, this can usually be achieved by giving FVIII two to three times a week.

An important advance in the treatment of Hemophilia A was the isolation of cDNA clones encoding the complete 2,351 amino acid sequence of human FVIII (see, Wood et al, Nature, 312: 330 (1984) and U.S. Pat. No. 4,757,006) and the provision of the human FVIII gene DNA sequence and recombinant methods for its production. FVIII products for the treatment of hemophilia include, but are not limited to: ADVATE® (Antihemophilic Factor (Recombinant), Plasma/Albumin-Free Method, rAHF-PFM), recombinant Antihemophilic Factor (BIOCLATE™, GENARC®, HELIXATE FS®, KOATE®, KOGENATE FS®, RECOMBINATE®): MONOCLATE-P®, purified preparation of Factor VIII:C, Antihemophilic Factor/von Willebrand Factor Complex (Human) HUMATE-P® and ALPHANATE®, Anti-hemophilic Factor/von Willebrand Factor Complex (Human); and HYATE C®, purified pig Factor VIII. ADVATE®, is produced in CHO-cells and manufactured by Baxter Healthcare Corporation. No human or animal plasma proteins or albumin are added in the cell culture process, purification, or final formulation of ADVATE®.

von Willebrand Factor exists in plasma in a series of multimer forms of a molecular weight of from 1x10⁶ to 20x10⁶ Dalton. VWF (Genbank Accession No. NP_000543) is a glycoprotein primarily formed in the endothelial cells of mammals and subsequently secreted into circulation. In this connection, starting from a polypeptide chain having a molecular weight of approximately 220 kD, a VWF dimer having a molecular weight of 550 kD is produced in the cells by the formation of several sulfur bonds. Further polymers of the VWF with increasing molecular weights, up to 20 million Dalton, are formed by the linking of VWF dimers. It is presumed that particularly the high-molecular VWF multimers have an essential importance in blood coagulation.

VWF syndrome manifests clinically when there is either an underproduction or an overproduction of VWF. Overproduction of VWF causes increased thrombosis (formation of a clot or thrombus inside a blood vessel, obstructing the flow of blood) while reduced levels of, or lack of, high-molecular forms of VWF causes increased bleeding and an increased bleeding time due to inhibition of platelet aggregation and wound closure.

A VWF deficiency may also cause a phenotypic hemophilia A since VWF is an essential component of functional Factor VIII. In these instances, the half-life of Factor VIII is reduced to such an extent that its function in the blood coagulation cascade is impaired. Patients suffering from von Willebrand disease (VWD) or VWF syndrome frequently exhibit a Factor VIII deficiency. In these patients, the reduced Factor VIII activity is not the consequence of a defect of the X chromosomal gene, but an indirect consequence of the quantitative and qualitative change of VWF in plasma. The differentiation between hemophilia A and VWD may normally be effected by measuring the VWF antigen or by determining the ristocetin-cofactor activity. Both the VWF antigen content and the ristocetin cofactor activity are lowered in most VWD patients, whereas they are normal in hemophilia A patients. VWF products for the treatment of VWF syndrome include, but are not limited to: HUMATE-P, IMMUNATE®, INNOBRAND®, and 8Y®, which are therapies comprising FVIII/VWF concentrate from plasma.

Factor VII (proconvertin), a serine protease enzyme, is one of the central proteins in the blood coagulation cascade (Genbank Accession No. NP_000122). The main role of Factor VII (FVII) is to initiate the process of coagulation in conjunction with tissue factor (TF). Upon vessel injury, TF is exposed to the blood and circulating Factor VII. Once bound to TF, FVII is activated to FVIIa by different proteases, among which are thrombin (Factor IIa), activated Factor X and the FVIIa-TF complex itself. Recombinant human Factor VIIa (NOVOSEVEN®) has been introduced for use in uncontrollable bleeding in hemophilia patients who have developed inhibitors against replacement coagulation factor.

Factor IX (FIX, Christmas Factor) (Genbank Accession No. NP_000124) is a serine protease that is inactive unless activated by Factor XIa or Factor VIIa (of the tissue factor pathway). When activated into Factor IXa, it acts by hydrolyzing an arginine-isoleucine bond in Factor X to form Factor Xa. Factor VIII is a required cofactor for FIX protease activity (Lowe GD, Br. J. Haematol. 115: 507-13, 2002). Deficiency of Factor IX causes hemophilia B or Christmas disease.

Additional blood factors include Factor II (thrombin) (Genbank Accession No. NP_000497), deficiencies of which cause thrombosis and dysprothrombinemia; Factor V, (Genbank Accession No. NP_000121), deficiencies of which cause hemorrhagic diathesis or a form of thrombophilia, which is known as activated protein C resistance, Factor XI (Genbank Accession No. NP_000119), deficiencies of which cause Rosenthal's syndrome (hemophilia C), and Factor XIII subunit A (Genbank Accession No. NP_000120) and subunit B (Genbank Accession No. NP_001985), deficiencies of which are characterized as a type I deficiency (deficiency in both the A and B subunits) and type II deficiency (deficiency in the A subunit alone), either of which can result in a lifelong bleeding tendency, defective wound healing, and habitual abortion.

### Polypeptide Analogs or Variants

Methods for preparing polypeptide fragments, variants or analogs are well-known in the art. Fragments of a polypeptide are prepared using methods including enzymatic cleavage (e.g., trypsin, chymotrypsin) and also using recombinant means to generate a polypeptide fragment having a specific amino acid sequence. Fragments are, in one aspect, generated to comprise a ligand binding domain, a receptor binding domain, a dimerization or multimerization domain, or any other identifiable domain known in the art.

Analogs are, in certain aspects, substantially homologous or substantially identical to the naturally-occurring polypeptide from which the analog is derived, and analogs contemplated by the invention are those which retain at least some of the biological activity of the naturally-occurring polypeptide as described previously.

Substitution analogs typically exchange one amino acid of the wild-type for another at one or more sites within the protein, and may be designed to modulate one or more properties of the polypeptide, such as stability against proteolytic cleavage, without the loss of other functions or properties. Substitutions of this kind are generally conservative. By "conservative amino acid substitution" is meant substitution of an amino acid with an amino acid having a side chain of a similar chemical character. Similar amino acids for making conservative substitutions include those having an acidic side chain (glutamic acid, aspartic acid); a basic side chain (arginine, lysine, histidine); a polar amide side chain (glutamine, asparagine); a hydrophobic, aliphatic side chain (leucine, isoleucine, valine, alanine, glycine); an aromatic side chain (phenylalanine, tryptophan, tyrosine); a small side chain (glycine, alanine, serine, threonine, methionine); or an aliphatic hydroxyl side chain (serine, threonine).

Polynucleotide analogs and fragments are readily generated by a worker of skill to encode biologically active fragments or analogs of the naturally-occurring molecule that possess the same or similar biological activity to the naturally occurring molecule. Routinely practiced methods include PCR techniques, enzymatic digestion of DNA encoding the protein molecule and ligation to heterologous polynucleotide sequences, and the like. For example, point mutagenesis, using PCR and other techniques well-known in the art, may be employed to identify with particularity which amino acid residues are important in particular activities associated with protein activity. Thus, one of skill in the art will be able to generate single base changes in the DNA strand to result in an altered codon and a missense mutation.

It is further contemplated that the protein or polypeptide is modified to make an analog which is a protein as described herein further comprising a second agent which is a polypeptide, i.e., a fusion protein. In one embodiment, the second agent which is a polypeptide is a cytokine, growth factor, blood factor, enzyme, chemokine, soluble cell-surface receptor, cell adhesion molecule, antibody, hormone, cytoskeletal protein, matrix protein, chaperone protein, structural protein, metabolic protein, and other therapeutic proteins known to those of skill in the art, or fragment or active domain of a protein described above or of any other type of protein known in the art. In a related embodiment, the second agent is a blood clotting factor such as Factor II, Factor III, Factor V, Factor VII, Factor VIIa, Factor VIII, Factor IX , Factor X, Factor XI, von Willebrand Factor and fibrinogen. The fusion protein contemplated is made by chemical or recombinant techniques well-known in the art.

Protein variants contemplated include polypeptides chemically modified by such techniques as ubiquitination, glycosylation, conjugation to therapeutic or diagnostic agents, labeling (*e.g*., with radionuclides or various enzymes), covalent polymer attachment such as PEGylation (derivatization with polyethylene glycol), introduction of non-hydrolyzable bonds, and insertion or substitution by chemical synthesis of amino acids such as ornithine, which do not normally occur in human proteins. Variants retain the binding properties of non-modified molecules of the invention.

Additional polypeptide variants useful in the methods of the present invention include polypeptides comprising polysialylated (PSA) moieties. Methods for preparing polysialylated polypeptide are described in United States Patent Publication 20060160948 and Saenko et al.. Haemophilia 12:42-51, 2006.

### Water Soluble Polymers

The specification contemplates chemically modified proteins or polypeptides, which have been linked to a chemical moiety that provides advantageous effects to production, viability of the protein or polypeptide. For example, nonspecific or site-specific (*e.g*., N-terminal) conjugation of water-soluble polymers, *e.g.,* PEG or PEO, to polypeptides is known in the art to improve half-life by potentially reducing immunogenicity, renal clearance, and/or improving protease resistance. Polypeptides may comprise water-soluble polymers, such as PEG, covalently linked to the peptide N- or C-terminus to increase the half-life and/or stability of the molecule.

Water-soluble polymers, including but not limited to, poly(alkylene glycols) such as polyethylene glycol (PEG), poly(propylene glycol) ("PPG"), copolymers of ethylene glycol and propylene glycol and the like, poly(oxyethylated polyol), poly(olefinic alcohol), poly(vinylpyrrolidone), poly(hydroxyalkylmethacrylamide), poly(hydroxyalkylmethacrylate), poly(saccharides), poly(α-hydroxy acid), poly(vinyl alcohol), polyphosphasphazene, polyoxazoline, poly(N-acryloylmorpholine), poly(alkylene oxide) polymers, poly(maleic acid), poly(DL-alanine), polysaccharides, such as carboxymethylcellulose, dextran, starch or starch derivatives, hyaluronic acid and chitin, poly(meth)acrylates, as well as polysialic acid (PSA), and combinations of any of the foregoing, are commonly conjugated to proteins or peptides to increase stability or size of a protein or peptide.

Macromolecule chemical modification is, in one aspect, performed in a nonspecific fashion (leading to mixtures of derivatized species) or in a site-specific fashion (based on wild-type macromolecule reactivity-directed derivatization and/or site-selective modification using a combination of site-directed mutagenesis and chemical modification) or, alternatively, using expressed protein ligation methods (Curr Opin Biotechnol. 13(4):297-303 (2002)).

The specification contemplates use of water-soluble polymers, e.g., PEG or PEO molecules that vary in type, conjugation, linkage and length. PEG-protein conjugates include but are not limited to linear or branched conjugates, polymer-proteins conjugates linked by NHS (N-hydroxysuccinimide)- or aldehyde-based chemistry, variants with a different chemical linkage between the water soluble polymer chain and conjugation site, and variants differing in lengths. The average molecular weight of the PEG may range from about 2 to 200 kiloDalton ("kDa"), from about 5 kDa to about 120 kDa, from about 10 kDa to about 100 kDa, from about 20 kDa to about 50 kDa, from about 10 kDa to about 25 kDa, from about 5 kDa to about 50 kDa, from about 5 kDa to about 10 kDa, or from about 2 kDa to 5 kDa.

In one reference aspect, the specification contemplates PEG-protein conjugates selected from the group consisting of linear PEG-protein conjugates that are NHS-conjugated and range in length from -(CH2-CH2-O)n-, where n = 10 to 2000, linear PEG-protein conjugates that are aldehyde-conjugated and range in length from-(CH2-CH2-O)n-, where n = 10 to 2000, two-arm branched and multi-arm PEG-protein conjugates that are NHS-conjugated and range in length, from 10 to 2000, and three-arm branched PEG-protein conjugates that are NHS-conjugated. The specification also contemplates PEG-protein conjugates that contain different chemical linkages, e.g., -CO(CH2)n-, and -(CH2)n- (where n = 1 to 5) between its conjugation site and the PEG chain. The specification further contemplates charged, anionic PEG-protein conjugates to reduce renal clearance, including but not limited to carboxylated, sulfated and phosphorylated compounds (anionic) (Caliceti, Adv Drug Deliv Rev 55:1261-77, 2003; Perlman, J Clin Endo Metab 88:3227-35, 2003; Pitkin, Antimicrb Ag Chemo 29: 440-44, 1986; Vehaskari, Kidney Intl 22:127-135, 1982). In a further embodiment, the peptide is optionally conjugated to a moiety including a bisphosphonate, carbohydrates, fatty acids, or further amino acids.

PEGs and PEOs include molecules with a distribution of molecular weights, i.e., polydisperse. The size distribution can be characterized statistically by its weight average molecular weight (Mw) and its number average molecular weight (Mn), the ratio of which is called the polydispersity index (Mw/Mn). Mw and Mn can be measured by mass spectroscopy. Most of the PEG-protein conjugates, particularly those conjugated to PEG larger than 1 KD, exhibit a range of molecular weights due to a polydisperse nature of the parent PEG molecule. For example, in case of mPEG2K (Sunbright ME-020HS, NOF), actual molecular masses are distributed over a range of 1.5 ∼ 3.0 KD with a polydispersity index of 1.036. Exceptions are proteins conjugated to MS(PEG)n (N=4, 8, 12 or 24, e.g., PEO4, PEO12)-based reagents (Pierce), which are specially prepared as monodisperse mixtures with discrete chain length and defined molecular weight.

To determine if the *in vivo* therapeutic half-life of a peptide would benefit from PEGylation, a variety of different PEG-protein conjugates are synthesized, characterized *in vitro* and in-vivo for pharmacokinetics.

Methods for preparing the PEGylated protein of the present specification generally comprise the steps of (a) reacting the protein of interest with polyethylene glycol under conditions whereby PEG becomes attached to the N-terminus/C-terminus of the protein, and (b) obtaining the reaction product(s). Because PEGylating a protein might significantly alter the intrinsic activity of the protein, different types of PEG are explored. The chemistry that can be used for PEGylation of protein includes the acylation of the primary amines of he protein using the NHS-ester of methoxy-PEG (O-[(N-Succinimidyloxycarbonyl)-methyl]-O'-methylpolyethylene glycol). Acylation with methoxy-PEG-NHS or methoxy-PEG-SPA results in an amide linkage that eliminates the charge from the original primary amine (also, Boc-PEG for C-terminus). Unlike ribosome protein synthesis, synthetic peptide synthesis proceeds from the C-terminus to the N-terminus. Therefore, Boc-PEG is one method (i.e. using tert-(B)utyl (o)xy (c)arbonyl (Boc, t-Boc) synthesis) to attach PEG to the C-terminus of the peptide (R. B. Merrifield (1963). "Solid Phase Peptide Synthesis. I. The Synthesis of a Tetrapeptide". J. Am. Chem. Soc. 85: 2149-2154). Alternatively, (F)luorenyl-(m)eth(o)xy-(c)arbonyl (FMOC) chemistry (Atherton, E.; Sheppard, R.C. (1989). Solid Phase peptide synthesis: a practical approach. Oxford, England: IRL Press.) is used because it does not require the hazardous use of hydrofluoric acid to remove side-chain protecting groups.

In the invention when the water soluble polymer is PSA, the average molecular weight of the PSA will range from about 2 to 80 kDa, from 5 to 60 kDa, from 10 to 40 kDa or from 15 to 25 kDa.

Exemplary stable linkers that can facilitate conjugation of the water soluble polymer to the polypeptide of interest include, but are not limited to, amide, amine, ether, carbamate, thiourea, urea, thiocarbamate, thiocarbonate, thioether, thioester, and dithiocarbamate linkages, such as ω,ω-aminoalkane, N-carboxyalkylmaleimide, or aminoalkanoic acids, maleimidobenzoyl sulfosuccinimide ester, glutaraldehyde, or succinic anhydride, N-carboxymethylmaleimide N,N'-disuccinimidyl oxalate and 1,1'-bis[6-(trifluoromethy)benzo-triazolyl] oxalate.

In other embodiments, the water soluble polymer is conjugated to the polypeptide using releasable, degradable or hydrolyzable linkers, according to the claims. A hydrolyzable bond is a relatively weak bond that reacts with water (i.e., is hydrolyzed) under physiological conditions. The tendency of a bond to hydrolyze in water will depend not only on the general type of linkage connecting two central atoms but also on the substituents attached to these central atoms. Methods of making conjugates comprising water soluble polymers having hydrolyzable linkers are described in US Patent 7,259,224 (Nektar Therapeutics) and US Patent 7,267,941 (Nektar Therapeutics and National Institutes of Health). For example, a PEG can be prepared having ester linkages in the polymer backbone that are subject to hydrolysis. This hydrolysis results in cleavage of the polymer into fragments of lower molecular weight. Appropriate hydrolytically unstable, releasable or degradable linkages include but are not limited to carboxylate ester, phosphate ester, anhydrides, acetals, ketals, acyloxyalkyl ether, imines, orthoesters, peptides and oligonucleotides, thioesters, thiolesters, and carbonates. Hydrolytically degradable linkages that may be contained within the polymer backbone include carbamate, carbonate, sulfate, and acyloxyalkyl ether linkages; imine linkages, resulting, for example, from reaction of an amine and an aldehyde (see, e.g., Ouchi et al., Polymer Preprints, 38(1):582-3 (1997)); carbamate, phosphate ester, hydrazone, acetal, ketal, or orthoester linkages, including acetone-bis-(N-maleimidoethyl)ketal linkers (MK).

The present methods provide for a substantially homogenous mixture of polymer-:protein conjugate. "Substantially homogenous" as used herein means that only polymer-protein conjugate molecules are observed. The polymer-protein conjugate has biological activity and the present "substantially homogenous" PEGylated protein preparations are those which are homogenous enough to display the advantages of a homogenous preparation, *e.g*., ease in clinical application in predictability of lot to lot pharmacokinetics.

The polymer molecules contemplated for use in the attachment methods described herein may be selected from among water-soluble polymers or a mixture thereof. The polymer may have a single reactive group, such as an active ester for acylation or an aldehyde for alkylation, so that the degree of polymerization may be controlled. The water soluble polymer, or mixture thereof if desired, may be selected from the group consisting of, for example, PEG, monomethoxy-PEG, PEO, dextran, starch or starch derivatives, poly-(N-vinyl pyrrolidone), propylene glycol homopolymers, fatty acids, a polypropylene oxide/ethylene oxide co-polymer, polyoxyethylated polyols (*e.g*., glycerol), HPMA, FLEXIMAR™, and polyvinyl alcohol, mono-(C1-C10)alkoxy-PEG, aryloxy-PEG, tresyl monomethoxy PEG, PEG propionaldehyde, bis-succinimidyl carbonate PEG, cellulose, other carbohydrate-based polymers, or mixtures thereof. The polymer selected should be water-soluble so that the protein to which it is attached does not precipitate in an aqueous environment, such as a physiological environment. The polymer may be branched or unbranched. Preferably, for therapeutic use of the end-product preparation, the polymer will be pharmaceutically acceptable. Methods for generating peptides comprising a PEG moiety are well-known in the art. See, for example, US Patent 5,824,784.

The term, PEG is meant to encompass any of the forms of PEG that have been used to derivatize other proteins, such as mono-(C1-C10) alkoxy- or aryloxy-polyethylene glycol. The PEG polymer may be branched or unbranched. Preferably, for therapeutic use of the end-product preparation, the polymer will be pharmaceutically acceptable. In one embodiment, the reactive aldehyde is PEG- propionaldehyde, which is water-stable, or mono-C1-C10 alkoxy or aryloxy derivatives thereof (see U.S. Patent No. 5,252,714).

The present specification contemplates several different linear PEG polymer lengths including but not limited to 10 to 2000 repeating units (-CH2-CH2-O-) or conjugates of two-armed branched PEG polymers. Further contemplated are NHS- or aldehyde-based PEG-(CH2CH2O)n, having from 12 to 50 units. In general, for the PEGylation reactions contemplated herein, the average molecular weight of the PEG moiety added is about 1 kDa to about 60kDa (the term "about" indicating +/-1 kDa). More preferably, the average molecular weight is about 10 - 40 kDa.

The invention provides modified proteins, namely FVIII, having a low degree of water soluble polymer conjugated to the protein. In a reference aspect, the low-PEGylated form of the protein is generated using a decreased molar excess of water soluble polymer to protein in the conjugation reaction. For example, typical methods to PEGylate a protein use a 61.8 M excess of PEG to protein of interest. It is contemplated that low PEGylated proteins as described herein are generated using a molar excess in the reaction that is less than that used in standard techniques. It is disclosed that the water soluble polymer may be polyethylene glycol (PEG). It is contemplated that the PEG is a linear or branched PEG, and may have the molecular weight and features as described herein.

Additionally, it is contemplated that the low-PEGylated protein described herein at comprises at least one and no more than 10 water soluble polymer moieties per blood factor molecule. In one reference aspect, the modified protein comprises at least 2, 3, 4, 5, 6, 7, 8, or 9 water soluble polymer moieties per protein molecule. In another reference aspect the modified protein comprises between 4 and 8 water soluble polymer moieties, inclusive (i.e., includes 4, 5, 6, 7 and 8 polymer moieties), per protein molecule. The modified protein is a blood factor. The modified blood factor may comprise between 1 and 4 water soluble polymer moieties, inclusive, per protein molecule. The modified blood factor may comprise between 4 and 6 water soluble polymer moieties, inclusive, per protein molecule. In still another embodiment the modified blood factor comprises 1 or 2 water soluble polymer moieties per protein molecule according to the claims.

As used herein, the term "between" when used in the context of numbers of water soluble polymers, e.g., "comprises between 4 and 8 water soluble polymers," is inclusive of the recited numbers and those numbers between the recited numbers. For example, between 4 and 8 water soluble polymers refers to 4, 5, 6, 7 and 8 water soluble polymers.

The blood factor is Factor VIII. In a still further embodiment, the blood factor molecule is human.

In another reference aspect the modified blood factor comprises between 4 and 8 PEG moieties, between 4 and 6 PEG moieties, or between 1 and 4 PEG moieties, inclusive, per Factor VIII molecule. In still another reference aspect the modified blood factor comprises 1 or 2 PEG moieties per Factor VIII molecule. It is contemplated that the PEG molecules are connected or conjugated to the blood factor via a stable, releasable or hydrolyzable linker.

The modified blood factor comprises 1 or 2 PSA moieties per Factor VIII molecule. It is contemplated that the PSA molecules are connected or conjugated to the blood factor via a stable, releasable or hydrolyzable linker.

In another reference aspect, the modified blood factor is FVIIa. In a related reference aspect, the modified blood factor comprises between 1 and 6 water soluble polymer moieties, inclusive, per FVIIa molecule. In some reference aspects, the modified blood factor comprises 1 or 2 water soluble polymer moieties per FVIIa molecule. In a related reference aspect, the water soluble polymer is selected from the group consisting of PEG or PSA. In certain embodiments, the polymers are connected via a stable, releasable or hydrolyzable linker.

In still other reference aspects, the modified blood factor is FIX. In one reference aspect, the modified blood factor comprises between 1 and 6 water soluble polymer moieties, inclusive, per FIX molecule. In some reference aspects, the modified blood factor comprises 1 or 2 water soluble polymer moieties per FIX molecule. In a related reference aspect, the water soluble polymer is selected from the group consisting of PEG or PSA. In one embodiment, the polymers are connected via a stable, releasable or hydrolyzable linker.

### Pharmaceutical Compositions

The present invention contemplates pharmaceutical compositions comprising effective amounts of protein or derivative products of the invention together with pharmaceutically acceptable diluents, stabilizers, preservatives, solubilizers, emulsifiers, adjuvants and/or carriers. Such compositions include diluents of various buffer content (*e*.*g*., Tris-HCl, phosphate), pH and ionic strength; additives such as detergents and solubilizing agents (*e.g*., Polysorbate 20, Polysorbate 80), anti-oxidants (*e.g*., ascorbic acid, sodium metabisulfite), preservatives (*e.g*., Thimerosol, benzyl alcohol) and bulking substances (*e.g*., lactose, mannitol); see, *e.g*., Remington's Pharmaceutical Sciences, 18th Edition (1990, Mack Publishing Co., Easton, Pa.) pages 1435:1712. An effective amount of active ingredient is a therapeutically, prophylactically, or diagnostically effective amount, which can be readily determined by a person skilled in the art by taking into consideration such factors as body weight,-age, and therapeutic goal.

The polymer-protein compositions of the present invention may also include a buffering agent to maintain the pH of the solution within a desired range. Preferred agents include sodium acetate, sodium phosphate, and sodium citrate. Mixtures of these buffering agents may also be used. The amount of buffering agent useful in the composition depends largely on the particular buffer used and the pH of the solution. For example, acetate is a more efficient buffer at pH 5 than pH 6 so less acetate may be used in a solution at pH 5 than at pH 6. The preferred pH range for the compositions of the present invention is pH 3.0-7.5.

The compositions of the present invention may further include an isotonicity-adjusting agent to render the solution isotonic and more compatible for injection. The most preferred agent is sodium chloride within a concentration range of 0-150 mM.

The methods described herein use pharmaceutical compositions comprising the molecules described above, together with one or more pharmaceutically acceptable excipients or vehicles, and optionally other therapeutic and/or prophylactic ingredients. Such excipients include liquids such as water, saline, glycerol, polyethylene glycol, hyaluronic acid, ethanol, cyclodextrins, modified cyclodextrins (*i*.*e*., sufobutyl ether cyclodextrins), *etc.* Suitable excipients for non-liquid formulations are also known to those of skill in the art.

Pharmaceutically acceptable salts can be used in the compositions of the present invention and include, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients and salts is available in Remington's Pharmaceutical Sciences, 18th Edition (Easton, Pennsylvania: Mack Publishing Company, 1990).

Additionally, auxiliary substances, such as wetting or emulsifying agents, biological buffering substances, surfactants, and the like, may be present in such vehicles. A biological buffer can be virtually any solution which is pharmacologically acceptable and which provides the formulation with the desired pH, *i.e*., a pH in the physiologically acceptable range. Examples of buffer solutions include saline, phosphate buffered saline, Tris buffered saline, Hank's buffered saline, and the like.

### Kits

As an additional aspect, the specification includes kits which comprise one or more compounds or compositions packaged in a manner which facilitates their use to practice methods of the invention. In one embodiment, such a kit includes a compound or composition described herein (e.g., a composition comprising a modified blood factor, such as low-PEGylated Factor VIII), packaged in a container such as a sealed bottle or vessel, with a label affixed to the container or included in the package that describes use of the compound or composition in practicing the method. Preferably, the compound or composition is packaged in a unit dosage form. The kit may further include a device suitable for administering the composition according to a specific route of administration. Preferably, the kit contains a label that describes use of the modified blood factor composition.

Additional aspects and details of the invention will be apparent from the following examples, which are intended to be illustrative.

### EXAMPLES Reference Example 1

### Synthesis of Low-PEGylated Factor VIII

Modification of blood clotting factors by addition of water soluble polymers has been carried out in order to prolong the half life and improve the stability of molecules that are administered as therapeutic proteins. However, a high degree of attachment of water soluble polymers can lead to greater toxicity in vivo. Therefore, in order to improve the efficacy of therapeutic molecules, experiments to reduce the degree of conjugation of water soluble polymers were performed.

Synthesis of PEGylated rFVIII containing an intact B-domain is described in US Patent Publication 20070244301 and International Patent Publication WO 2007/126808. This PEGylated rFVIII containing an intact B-domain showed improved in vitro and in vivo characteristics under experimental conditions, and resulted in at least a partially PEGylated light chain (A3-C1-C2) of the rFVIII molecule.

However, a chemical process leading to a relatively high degree of modification of the therapeutic protein is not economical due to the required high amounts of reagents. In addition high PEGylation degrees lead to an increased toxicological risk due to high amounts of the polymer and the linker. Thus, in order to generate a molecule having a lower degree of PEG moieties, the reagent concentration in the preparation process was reduced and different PEG-rFVIII were prepared by reducing the reagent concentration from 61.8 M excess (standard) to 30 M, 25 M, 20 M and 15 M excess, respectively.

PEGylation variants described in the art have varying release characteristics as well as different molecular weights of the PEG chains. See e.g., WO 2008082669 and US20080234193 (Nektar Therapeutics and Baxter Healthcare). One PEGylated rFVIII variant having a branched (Lys 20K branch long) with a molecular weight of the PEG chain of 20 kD with a long release characteristic was prepared by use of a 61.8 M excess of a releasable PEG reagent. This PEGylated rFVIII was selected as a lead candidate to modify the degree of PEGylation due to the molecules measured in vitro and in vivo data. For the initial PEGylated rFVIII candidate, a PEGylation degree of 11.1 PEG/rFVIII (mol/mol) was measured by use of HPLC methods as described in the art (see e.g., Chen et al., Bioconjug Chem. 18:371-8, 2007) when the conjugate was prepared using a 61.8 M excess of PEG.

The process for PEGylation of rFVIII was as follows: PEGylation of rFVIII for 2h at R.T. at pH 7.2 +/- 0.2 (c = 2 mg/ml); Reagent concentration (3.9 mg reagent/mg protein - 61.8 M excess); Stopping/quenching of the reaction by addition of glycine; Purification on Q-Sepharose HP. Elution with ∼ 0.5 M NaCl; UF/DF and final formulation of PEGylated molecule. The low-PEG conjugates were prepared as above using the indicated molar excess of the PEG polymer. PEG-rFVIII samples used were as follows: 15 M excess: VIEHLUFB08007PHR; 20 M excess: VIEHLUFB08016PHR; 25 M excess: VIEHLUFB08017PHR; 30 M excess: VIEHLUFB08008PHR, VIEHLUFB08009PHR, VIEHLUFB07029PHR; 61.8 M excess (standard protocol in the art) and native FVIII: VIEHLUFB08018PHR, ORHLUFB07016PHR, ORHLUFB07017PHR, ORHLUFB08001PHR, ORHLUFB08002PHR.

For the resynthesized candidate a degree of 8 PEG/rFVIII was determined. The low-PEG-FVIII were further characterized in vitro and in vivo as described in the examples below.

### Reference Example 2

### Analysis of low PEGylated Blood Factor Molecules In Vitro

In one aspect, the low PEG samples were analyzed for the molecular weight and general structure of the PEG-FVIII molecule, as well as for the specific activity of the PEG-conjugated FVIII molecule. SDS-PAGE analysis of the PEG-FVIII structure was carried out as in WO 2007/126808. Briefly, native rFVIII was characterized by SDS PAGE under reducing conditions by using a 4-12% polyacrylamide gradient gel obtained from Invitrogen (Carlsbad, California, USA) according to the instructions of the manufacturer. As molecular weight markers (MW) Precision Plus markers (10 kD - 250 kD) obtained from Bio- Rad (Hercules, CA, USA) were used. Then the proteins were transferred on a PVDF membrane obtained from Bio-Rad (Hercules, CA, USA) by electroblotting and subsequently incubated with a polyclonal sheep anti human FVIII:C antibody obtained from Cedarlane (Hornby, Ontario, Canada). The last steps of the immunostaining procedure were the incubation with an alkaline phosphatase (ALP) conjugated anti-sheep antibody obtained from Accurate (Westbury, NY, USA) followed by the final visualization by use of an ALP substrate kit (Bio-Rad, Hercules, CA, USA).

Additionally, the specific activity of the FVIII molecule was assayed as described in WO 2007/126808, using the FVIII chromogenic assay (Rosen S, Scand J Haematol 33:(Suppl 40):139-45, 1984). The method is based on Ph. Eur. 5th edition (5.05) 2.7.4 Assay of Blood Coagulation Factor VIII. A sample, containing Factor VIII is mixed with thrombin, activated Factor IX (FIXa), phospholipids and Factor X (FX) in a buffer containing calcium. FVIII is activated by thrombin and subsequently forms a complex with phospholipids, FIXa and calcium ions. This complex activates Factor X to Factor Xa, which in turn cleaves the chromogenic substrate FXa-1 (AcOH*CH3OCO-D-CHA-Gly-Arg-pNA). The time course of para-nitroaniline (pNA) released is measured with a micro plate reader at 405 nm. The slope of the reaction is proportional to the Factor VIII concentration in the sample. The FVIII antigen value was measured by use of an ELISA system commercially available (Cedarlane, Hornby, Ontario, Canada) with minor modifications. From these values the ratios FVIII chromogen/FVIII antigen were calculated. The protein content in the preparations was determined by measuring the optical density at 280nm. From these data the protein content was calculated.

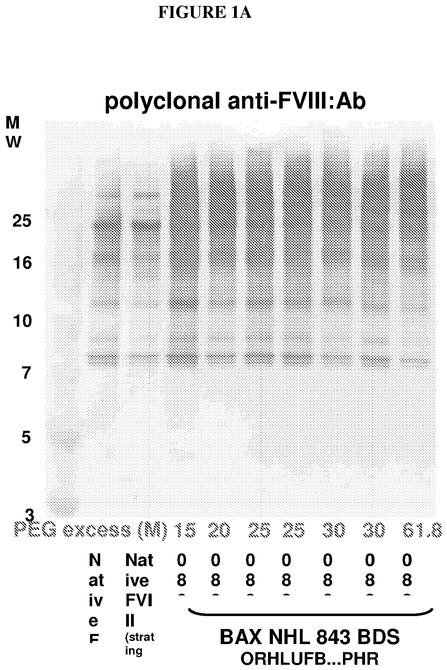

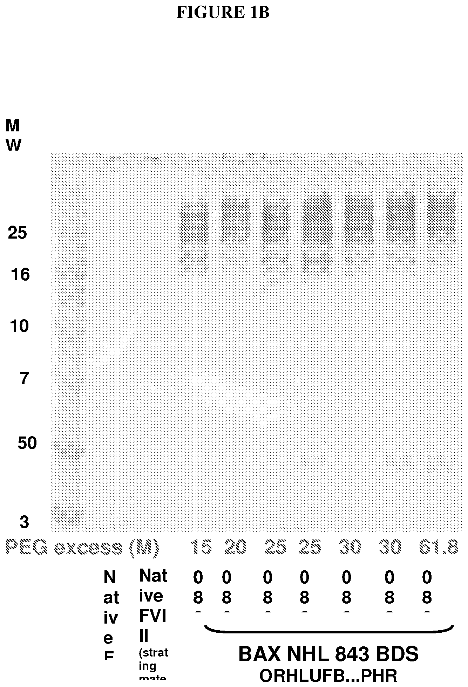

SDS-PAGE results are shown in Figure 1. SDS-PAGE evaluation of the FVIII content using FVIII specific antibodies demonstrates that FVIII combined with a lower molar excess of PEG molecules (15, 20, 25 and 30 M excess) produced lower molecular weight molecules compared to the native FVIII, but the FVIII molecules detected are similar to those which appear when a higher excess of PEG molecules (61.8 M excess) are used. Probing of the SDS-PAGE with a PEG-specific antibody detected higher molecular weight species of the PEG-FVIII in all PEG samples tested.

Analysis of the specific activity of low PEGylated FVIII (and relevant product data) is shown in Table 1. These results show that the specific activity of the low-PEGylated FVIII is more similar to native FVIII than PEG-FVIII prepared using the high molar excess. For example, the PEG-FVIII sample prepared using only a 15M excess of PEG demonstrated a specific activity of 2221 IU/mg compared to native FVIII specific activity of 3706 IU/mg. In contrast, samples prepared using the common PEGylation protocol, having a 61.8M excess of PEG, showed a specific activity from as high as 398 IU/mg to as low as 104 IU/mg. Thus, PEG-FVIII prepared using 15M excess PEG exhibited approximately 5.5 times greater activity than the highest activity of a PEG-FVIII prepared using standard protocols (61.8 M excess). Similarly, FVIII prepared with 20M excess PEG showed activity at least 3.8 times greater than FVIII prepared using standard protocols, 25M excess PEG-FVIII molecules exhibited activity at least 3.2 times greater than standard preparation protocols and 30M excess PEG-FVIII (969 IU/ml) showed at least 2.4 times greater than standard (61.8M excess PEG) preparation protocols.

These results illustrate that FVIII proteins PEGylated using a low molar excess of PEG to FVIII ratio results in low-PEG-FVIII having a biological activity nearing that of the native FVIII protein compared to PEG-FVIII prepared using a high molar excess PEG, which show a specific activity approximately 9 times lower than native FVIII. The increased specific activity and lower reduced number of PEG molecules provides a more efficient therapeutic molecule having reduced possibility of toxic side effects.

### Reference Example 3

### Pharmacokinetics of low PEGylated molecules in vivo

In order to determine the pharmacokinetics of the low-PEGylated rFVIII in vivo, a FVIII deficient knock out mouse model was used. FVIII deficient mice as described in Bi et al. (Nat Genet 1995;10:119-21) were used as a model of severe human hemophilia A.

Mice (n=6) received a bolus injection via the tail vein with either low-PEG-FVIII prepared according to Example 1 or native rFVIII in a dose of 20-30 µg/kg bodyweight. PEG-rFVIII samples used were as follows: rFVIIIPEGH07001FC (8.5 PEG degree - mol/mol, bound PEG) at 297 µg/kg; VIEHLUFB07029PHR (7.9 PEG degree -mol/mol, bound PEG) at 144 µg/kg; VIEHLUFB08007PHR (4.4 PEG degree -mol/mol, bound PEG) at 66 µg/kg. Citrate plasma by heart puncture after anesthesia was prepared from the respective groups, at 5 minutes, 3, 6, 9, 16, 24, and 32 hours, and in some cases at 48, 56 and 72 hours, intervals after injection. FVIII activity levels were measured in plasma samples. Half-life calculation was performed with MicroMath Scientist, model 1 from pharmacokinetic library (MicroMath, Saint Louis, MO, USA).

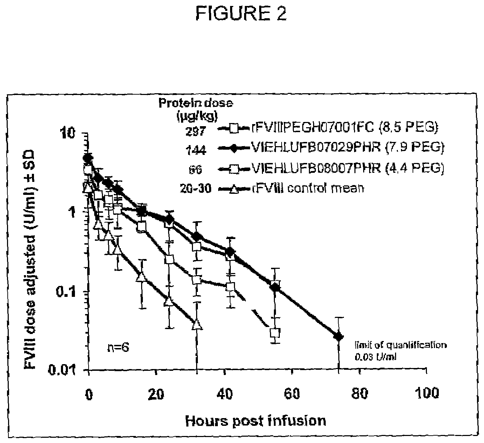

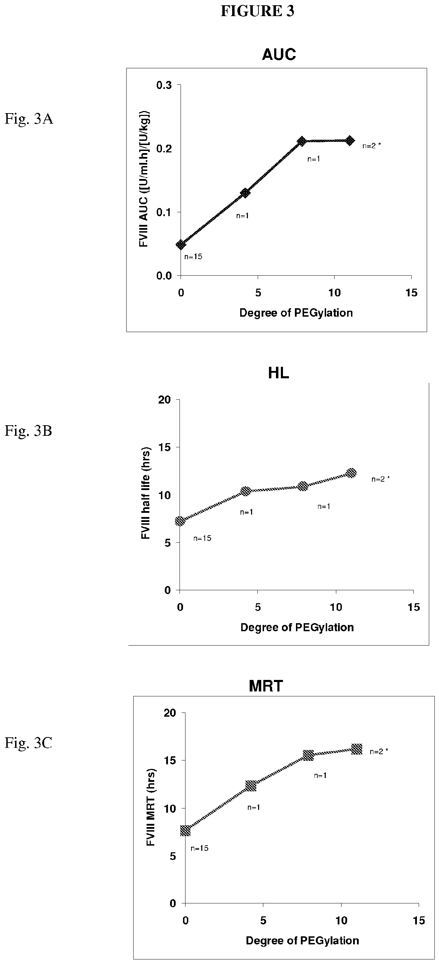

The results of this experiment are summarized in Figures 2 and 3 and Table 2. The results show that the terminal half-life is similar for PEG-rFVIII with PEGylation degrees between 4.4 and 8.5 (Figure 3 and Table 2). Table 2 illustrates that the half-life (HL) and mean residence time (MRT) of the low PEGylated rFVIII is increased compared to the native FVIII. Additionally, the area under the curve (AUC) is increased in the low PEGylated FVIII compared to native FVIII, indicating the improved pharmacokinetics of low PEGylated FVIII variant in comparison to native rFVIII. In addition, the protein load to achieve the desired FVIII activity dose is lower with PEG-rFVIII variants with lower PEGylation degree.

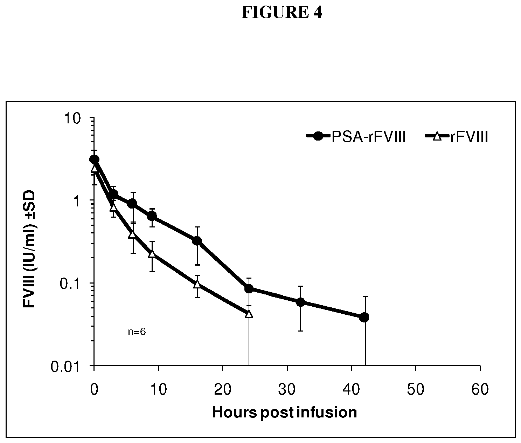

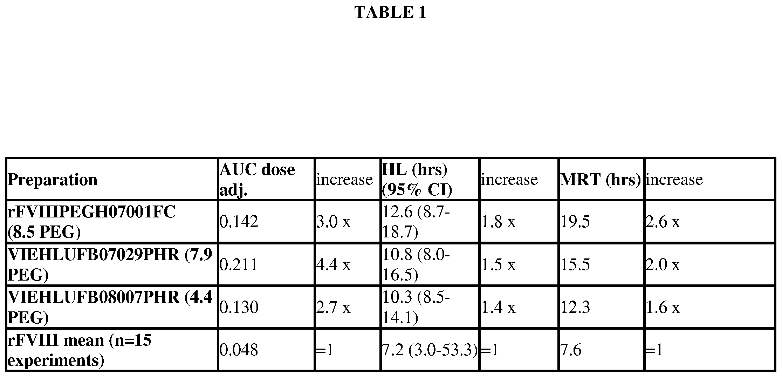

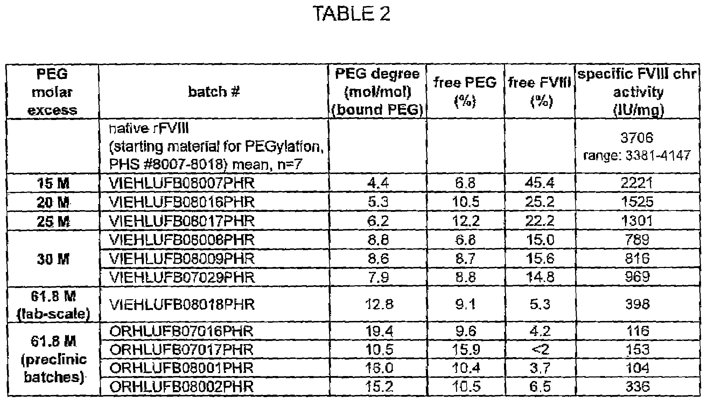

A plot of the AUC, half-life and MRT data against the degree of PEGylation (Figure 4) shows that there is a linear correlation of AUC and MRT with the degree of PEGylation up to approximately 8 PEG/FVIII, and there is no further increase with higher degrees of PEGylation. The terminal half-life increases slightly when the number of PEG molecules per FVIII is greater than 5 PEG/FVIII.

In summary, the data suggests that low PEGylated rFVIII variants prepared using 15M, 20M, 25M and 30M excess PEG, have improved *in vivo* and *in vitro* properties compared with PEG- rFVIII prepared according to the standard process (61.8M excess PEG). The activity of the low-PEGylated form of FVIII is more similar to that of the native molecule than previous high-PEG-FVIII preparations, suggesting that the low-PEG blood factors can be used at a lower dose during therapy, thereby reducing the possibility of developing neutralizing antibodies against the blood factor and reducing the toxicity of the composition.

### Reference Example 4

### Preparation of Low PEGylated rFIX

It is contemplated that other blood factor proteins are conjugated to water soluble polymers as described herein. For example, recombinant FIX (rFIX) is PEGylated by use of a linear 20 kD PEGylation reagent containing an NHS group. An example of this type of reagent is the SUNBRIGHT ® ME series from NOF (NOF Corp., Tokyo, Japan). rFIX is PEGylated at pH 7.4 in Hepes buffer (20 mM Hepes, 150 mM NaCl) at a protein concentration of 2 mg/ml and a reagent concentration of 5 mg/ml. The PEGylation reaction is carried out at RT for 2 hours under gentle shaking. Then the reaction is stopped by addition of glycine (final concentration: 10 mM) and incubation for 1 hour at room temperature. The mixture is then applied to a Q - Sepharose HP column (GE-Healthcare, Uppsala, Sweden) for purification and separation of mono PEGylated rFIX, containing only one PEG residue, from native rFIX and traces of di- and tri-PEGylated rFIX. Finally, the fractions containing mono PEGylated rFIX are collected and subjected to ultrafiltration/diafiltration (UF/DF) using a 30 kD membrane made of regenerated cellulose (Millipore).

### Reference Example 5

### Preparation of Low PEGylated rFVIIa

Recombinant FVIIa (rFVIIa) is PEGylated by use of a linear 20 kD PEGylation reagent containing an NHS group. An example of this type of reagent is the SUNBRIGHT ® GS series from NOF (NOF Corp., Tokyo, Japan). rFVIIa is PEGylated at pH 7.4 in Hepes buffer (20 mM Hepes, 150 mM NaCl) at a protein concentration of 2 mg/ml and a reagent concentration of 5 mg/ml. The PEGylation reaction is carried out at RT for 2 hours under gentle shaking. The reaction is stopped by addition of glycine (final concentration: 10 mM) and incubation for 1 hour at room temperature. Finally the PEG-rFVIIa conjugate is purified by ion-exchange chromatography on Q-Sepharose FF (GE Healthcare). The solution is loaded onto the column, which is preequilibrated with 20 mM Hepes buffer containing 1 mM CaCl₂, pH 7.4 (loading capacity: 1.5 mg protein/ml gel). The conjugate is eluted with 20 mM Hepes buffer containing 1 mM CaCl₂ and 500 mM sodium chloride. The eluate contains predominantly mono PEGylated rFVIIa. Finally the eluate is concentrated by UF/DF using a 30 kD membrane made of Polyethersulfone (Millipore).

### Example 6

### Preparation of Low Polysialylated rFVIII

In addition to PEGylation, blood factor proteins are conjugated to other water soluble polymers.

rFVIII was polysialylated by reductive amination using oxidized polysialic acid (PSA) with a narrow size distribution (PD ≤ 1.1) and a MW of 20 kD, which was obtained from the Serum Institute of India (Pune, India).

The conjugation of PSA with rFVIII was carried out at + 4°C in a cold room. The conjugation was performed with a rFVIII concentration of 2 mg/ml, and with a 200 fold molar excess of oxidized PSA. The PSA was dissolved in Hepes buffer (50 mM Hepes buffer, 5 mM CaCl₂, 350mM NaCl, pH 7.4) to give a final concentration of 200 mg PSA /ml. The PSA solution was added to the rFVIII solution and the required amount of NaCNBH₃ was added in a solution of 80 mg/ml in Hepes buffer, pH 7.4 to give a final concentration of 50 mM. The reaction mixture was gently mixed and the pH adjusted to 7.4 by drop-wise addition of 0.5 M NaOH to pH 7.4. The reaction mixture was gently shaken in the dark for 16 hours at +4°C in a cold room. The conjugate was then purified by Hydrophobic Interaction Chromatography (HIC) on Phenyl - Sepharose FF (GE Healthcare). After the chemical reaction the reaction mixture was diluted with 8 M NH₄Ac in Hepes buffer (50 mM Hepes, 350 mM NaCl, 5 mM CaCl₂, pH 6.9) to give a final concentration of 2.5 M and pH was corrected by addition of 0.5M NaOH to pH 6.9. Then the sample was loaded onto the HIC column. The column was washed with approximately 2.5 column volumes (CV) of wash buffer (2.5 M NH₄Ac in 50 mM Hepes, 350 mM NaCl, 5 mM CaCl₂, pH 6.9) followed by washing with approximately 10 CV wash buffer (3M NaCl in 50 mM Hepes, 5mM CaCl₂; pH 6.9). The column was eluted with 6 CV of elution buffer (50 mM Hepes, 5 mM CaCl₂, pH 7.4). The conjugate-containing fraction was subjected to UF/DF using a 30kD membrane (regenerated cellulose/Millipore).

### Example 7

### In vitro and in vivo Characterization of Low Polysialylated rFVIII

The PSA-rFVIII conjugate having a low degree of polysialylation prepared by reductive amination according to Example 6 was assayed for protein activity in vitro and in vivo.

The PSA-rFVIII preparation was analytically characterized by measuring the protein content (BCA assay) and the FVIII chromogenic activity. A specific activity of 2469 IU/mg was calculated for this preparation. This are 44 % as compared to the rFVIII starting material. The degree of polysialyalation was determined by use of the Resorcinol assay (Svennerholm L, Biochim Biophys Acta 24:604-11;1957). A polysialylation degree of 2.1 PSA molecules / monomer FVIII was measured.

The PSA-rFVIII was also used for pharmacokinetic (PK) studies in hemophilic mice. Groups of 6 hemophilic mice received a bolus injection via the tail vein in a dose of 200 IU FVIII /kg bodyweight. Citrate plasma by heart puncture after anesthesia was prepared from the respective groups, at 5 minutes, 3, 6, 9, 16, 24, 32 hours and in some cases at 42 hours after injection. FVIII activity levels were measured in plasma samples. Half-life and area under the curve calculation (AUC) was performed with MS Excel. The results of this experiment are illustrated in Figure 4. For this elimination curve a dose adjusted AUC of 0.054 (IU x h/ml)/(IU/kg) for FVIII and 0.076 (IU x h/ml)/(IU/kg) for the PSA-rFVIII conjugate was measured. The results show that the PSA-rFVIII circulates longer than native rFVIII and the corresponding AUC of PSA-rFVIII is increased by a factor of 1.4.

### Reference Example 8

### Preparation of Low Polysialylated rFIX

Recombinant FIX (rFIX) is polysialylated by reductive amination using oxidized Polysialic acid (PSA) with a narrow size distribution (PD ≤ 1.1) and a MW of 20 kD, which can be obtained from the Serum Institute of India (Pune, India).

The conjugation of PSA with rFIX is carried out at + 4°C in a cold room. The conjugation is performed using a rFIX concentration of 2 mg/ml, and with a 160 fold molar excess of oxidized PSA. The PSA is dissolved in Hepes buffer (50 mM Hepes buffer, 5 mM CaCl₂, 350mM NaCl, pH 7.4) to give a final concentration of 200 mg PSA/ml. The PSA solution is added to the rFIX solution and the required amount of NaCNBH₃ is added in a solution of 80 mg/ml in Hepes buffer, pH 7.4 to give a final concentration of 50 mM and pH of obtained solution is adjusted by drop-wise addition of 0.5M NaOH to pH 7.4. The reaction mixture is gently shaken in the dark for 16 hours at +4°C in a cold room. Subsequently the conjugate is purified by Hydrophobic Interaction Chromatography (HIC) on Butyl Sepharose FF (GE Healthcare). After the chemical reaction, the reaction mixture is diluted with 5 M NaCl in Hepes buffer (50 mM Hepes, 5 mM CaCl₂, pH 6.9) to give a final concentration of 3 M and the pH is adjusted to pH 6.9 using 0.5 M NaOH. The sample is loaded onto the HIC column and washed with 10 column volumes (CV) of equilibration buffer (3.0 M NaCl in 50 mM Hepes, 5 mM CaCl₂, pH 6.9). Subsequently the PSA-rFIX conjugate is eluted within 6 CV of elution buffer (50 mM Hepes, 5 mM CaCl₂, pH 7.4). The conjugate-containing fraction is subjected to UF/DF using a 30kD membrane (regenerated cellulose/Millipore). It is expected that the preparation contains predominantly mono- and di-PSAylated rFIX.

### Reference Example 9

### Preparation of Low Polysialylated rFVIIa

rFVIIa is polysialylated by reductive amination using oxidized polysialic acid (PSA) with a narrow size distribution (PD ≤ 1.1) and a MW of 20 kD, which can be obtained from the Serum Institute of India (Pune, India).

The conjugation of PSA with rFVIIa is carried out at + 4°C in a cold room. The conjugation reaction is performed using a rFVIIa concentration of 2 mg/ml, and with a 125 fold molar excess of oxidized PSA. The PSA is dissolved in Hepes buffer (50 mM Hepes buffer, 5 mM CaCl₂, 350mM NaCl, pH 7.4) and the pH is adjusted to 7.4 by drop-wise addition of 2 M NaOH to give a final concentration of 150 mg/ml. The PSA solution is added to the rFVIIa solution and the required amount of NaCNBH₃ is added in a solution of 80 mg/ml in Hepes buffer, pH 7.4 to give a final concentration of 50 mM. The reaction mixture is gently mixed and the pH is adjusted to 7.4 again. The reaction mixture is gently shaken in the dark for 16 hours. Subsequently the conjugate is purified by Hydrophobic Interaction Chromatography (HIC) on Butyl Sepharose FF (GE Healthcare). After the chemical reaction, the reaction mixture is diluted with 5 M NaCl in Hepes buffer (50 mM Hepes, 5 mM CaCl₂, pH 6.9) to give a final concentration of 3 M. Then the sample is loaded onto the HIC column and washed with 10 column volumes (CV) of equilibration buffer (3.0 M NaCl in 50 mM Hepes, 5 mM CaCl₂, pH 7.4). Subsequently the PSA-rFVIIa conjugate is eluted with 10 CV of elution buffer (50 mM Hepes, 5 mM CaCl₂, pH 7.4). The conjugate containing fractions are combined and subjected to UF/DF using a 30kD membrane (regenerated cellulose/Millipore). Finally the eluate is concentrated by UF/DF using a 30 kD membrane made of Polyethersulfone (Millipore). It is expected that the preparation contains predominantly mono and di-PSAylated rFVIIa.

### Example 10

### Treatment of Blood Clotting Disorders Using Blood Factors Modified With Low Degree of Water Soluble Polymer

Subjects having a deficiency in a blood clotting factor are treated with modified blood factor compositions as described herein. Administration of modified blood factor(s) having a low degree of water soluble polymer in animal models of blood clotting disorders and using protocols known in the art to treat humans suffering from blood disorders provides the basis for administering subjects the modified blood factor(s) described herein alone or in combination with other therapeutic agents, e.g,. chemotherapeutic or radiotherapeutic agents, cytokines, growth factors, and other commonly used therapeutics.

For example, hemophilia A patients having a deficiency in FVIII are treated with low-PEGylated FVIII at therapeutically effective doses, as is readily determined by the treating physician. See for example, Di Paola et al., Haemophilia. 13:124-30, 2007, which describes administration and comparison of two different preparations of replacement FVIII to patients with severe hemophilia.

Hemophilia patients who may or may not have a deficiency in FVIII protein (e.g., hemophilia B or C patients) are also treated with other modified blood factors such as modified VWF, FVII, FIX, FXI or those appropriate for the disease state. See, for example, Konkle et al., J Thromb Haemost. 5:1904-13, 2007, which describes treatment of hemophilia patients who developed inhibitors against FVIII and FIX with purified FVIIa.

Purified VWF has been used to treat patients suffering von Willebrands disease (Majumdar et al., Blood Coagul Fibrinolysis. 4:1035-7, 1993). Modified VWF having a low degree of water soluble polymer as described herein is used in regimens known to those of skill in the art to treat patients who would benefit from replacement VWF.

Additionally, other blood clotting disorders known in the art, e.g, Factor X deficiency, Factor VII deficiency, Alexander's disease, and Factor XIII deficiency may be treated with therapeutically effective doses of the appropriate modified blood factor(s).

Administration of the modified blood factors may last 1-24 hours, or longer, and is amenable to optimization using routine experimentation. The modified blood factor may also be given for a duration not requiring extended treatment. Additionally, modified blood factor composition may be administered daily, weekly, bi-weekly, or at other effective frequencies, as would be determinable by one of ordinary skill in the art.

It is contemplated that a modified blood factor is administered to patients in combination with other therapeutics, such as with other chemotherapeutic or radiotherapeutic agents, or with growth factors or cytokines. When given in combination with another agent, the amount of modified blood factor may be reduced accordingly. Second agents are administered in an amount determined to be safe and effective at ameliorating human disease.

It is contemplated that cytokines or growth factors, and chemotherapeutic agents or radiotherapeutic agents are administered in the same formulation as modified blood factor and given simultaneously. Alternatively, the agents may also be administered in a separate formulation and still be administered concurrently with modified blood factor. As used herein, concurrently refers to agents given within 30 minutes of each other. The second agent may also be administered prior to administration of modified blood factor. Prior administration refers to administration of the agent within the range of one week prior to modified blood factor treatment up to 30 minutes before administration of modified blood factor. It is further contemplated that the second agent is administered subsequent to administration of modified blood factor. Subsequent administration is meant to describe administration from 30 minutes after modified blood factor treatment up to one week after modified blood factor administration. Modified blood factor compositions may also be administered in conjunction with a regimen of radiation therapy in a subject having a blood clotting disorder and a form of cancer, treatment being carried out as prescribed by a treating physician.

Numerous modifications and variations in the invention as set forth in the above illustrative examples are expected to occur to those skilled in the art. Consequently only such limitations as appear in the appended claims should be placed on the invention.

## Claims

1. A chemically-modified Factor VIII (FVIII) molecule comprising a recombinant FVIII molecule and 1 or 2 polysialic acid (PSA) moieties per FVIII molecule, wherein the PSA moieties are attached to the FVIII molecule through reductive amination.

2. The chemically-modified FVIII molecule of claim 1 wherein the PSA moieties are attached via releasable or hydrolyzable linker, or via a stable linker.

3. The chemically-modified FVIII molecule of Claim 1, wherein the molecular weight of the PSA is in the range of from 2 to 80 kDa, preferably in the range of from 5 to 60 kDa, more preferably in the range of from 10 to 40 kDa or most preferably in the range of from 15 to 25 kDa.

4. A pharmaceutical composition comprising the modified FVIII molecule of any one of claims 1 to 3.

5. A chemically-modified FVIII molecule according to any one of Claims 1 to 3 for use in the treatment of a subject suffering from a blood clotting disorder.

6. The chemically-modified FVIII molecule for the use according to Claim 5, wherein the blood clotting disorder is selected from the group consisting of hemophilia A and von Willebrand Syndrome.

7. The chemically-modified FVIII molecule for the use according to Claim 5, wherein the blood clotting disorder is hemophilia A.

## Patentansprüche

1. Chemisch-modifiziertes Faktor VIII (FVIII)-Molekül, welches ein rekombinantes FVIII-Molekül und 1 oder 2 Polysialinsäure (PSA)-Reste pro FVIII-Molekül umfasst, wobei die PSA-Reste an das FVIII-Molekül durch reduktive Aminierung angefügt werden.

2. Chemisch-modifiziertes FVIII-Molekül gemäß Anspruch 1, wobei die PSA-Reste durch einen lösbaren oder hydrolysierbaren Linker, oder einen stabilen Linker angefügt werden.

3. Chemisch-modifiziertes FVIII-Molekül gemäß Anspruch 1, wobei das Molekulargewicht des PSA im Bereich von 2 bis 80 kDa liegt, bevorzugt im Bereich von 5 bis 60 kDa liegt, weiter bevorzugt im Bereich von 10 bis 40 kDa liegt, oder besonders bevorzugt im Bereich von 15 bis 25 kDa liegt.

4. Pharmazeutische Zusammensetzung, welche das modifizierte FVIII-Molekül gemäß irgendeinem der Ansprüche 1 bis 3 umfasst.

5. Chemisch-modifiziertes FVIII-Molekül gemäß irgendeinem der Ansprüche 1 bis 3 für die Verwendung in der Behandlung eines Subjekts, welches an einer Blutgerinnungsstörung leidet.

6. Chemisch-modifiziertes FVIII-Molekül für die Verwendung gemäß Anspruch 5, wobei die Blutgerinnungsstörung aus der Gruppe, bestehend aus Hämophilie A und von-Willebrand-Syndrom, ausgewählt wird.

7. Chemisch-modifiziertes FVIII-Molekül für die Verwendung gemäß Anspruch 5, wobei die Blutgerinnungsstörung Hämophilie A ist.

## Revendications

1. Molécule de facteur VIII modifiée chimiquement (FVIII) comprenant une molécule de FVIII recombinant et 1 ou 2 groupements d'acide polysialique (PSA) par molécule de FVIII, dans laquelle les groupements de PSA sont attachés à la molécule de FVIII par amination réductrice.

2. Molécule de FVIII modifiée chimiquement selon la revendication 1, dans laquelle les groupements de PSA sont attachés via un lieur libérable ou hydrolysable, ou via un lieur stable.

3. Molécule de FVIII modifiée chimiquement selon la revendication 1, dans laquelle le poids moléculaire du PSA est compris entre 2 et 80 kDa, de préférence entre 5 et 60 kDa, plus préférablement entre 10 et 40 kDa ou le plus préférablement entre 15 à 25 kDa.

4. Composition pharmaceutique comprenant la molécule de FVIII modifiée selon l'une quelconque des revendications 1 à 3.

5. Molécule de FVIII modifiée chimiquement selon l'une quelconque des revendications 1 à 3 pour une utilisation dans le traitement d'un sujet souffrant d'un trouble de la coagulation du sang.

6. Molécule de FVIII modifiée chimiquement pour l'utilisation selon la revendication 5, dans laquelle le trouble de la coagulation du sang est sélectionné à partir du groupe constitué par l'hémophilie A et le syndrome de von Willebrand.

7. Molécule de FVIII modifiée chimiquement pour l'utilisation selon la revendication 5, dans laquelle le trouble de la coagulation du sang est l'hémophilie A.
